# EUROPEAN PATENT APPLICATION

(11) **EP 4 759 243 A1**
(43) Date of publication of application: **17.06.2026**
(21) Application number: 25222112.2
(22) Date of filing: 10.12.2025
(51) Int. Cl.: A61B 8/00, A61B 6/00, A61B 6/46

(54) **MEDICAL APPARATUS**

(30) Priority: 13.12.2024 JP 2024218425
(71) Applicant: FUJIFILM Corporation, Tokyo 106-8620 (JP)
(72) Inventor: KANAI, Keno, Kanagawa (JP)
(74) Representative: Meissner Bolte Partnerschaft mbB

(57) **Abstract**

Provided is a medical apparatus capable of expanding a display region to display more pieces of biological information.

A display region 20 is provided on at least a part of a front portion. A length of the display region in a longitudinal direction is longer than a length of the display region in a width direction, and a lower end of the display region is provided below a grip portion of a handle in the vertical direction. The handle is attached to a side end portion or a back portion, and an attachment position of the handle is fixed relative to the display region.

## Description

### BACKGROUND OF THE INVENTION

### 1. Field of the Invention

The present disclosure relates to a medical apparatus that primarily acquires and displays biological information.

### 2. Description of the Related Art

In the medical field, from the viewpoint of improving the accuracy of a diagnosis, combinations of a plurality of pieces of biological information are increasingly used for an examination, a diagnosis, and the like. As disclosed in JP1994-19433A (JP-H6-19433A) and JP1997-289975A (JP-H9-289975A), these pieces of biological information are displayed on a plurality of displays connected vertically or horizontally.

### SUMMARY OF THE INVENTION

As described above, in a case where a plurality of pieces of biological information are displayed on a display, it is considered to expand a display region of the display. However, in actual medical settings, the extension of the display region may be subjected to constraints. In many medical apparatuses, a handle to be gripped by a user during movement is provided at around a height of a user's waist. However, in a case where an attempt is made to expand the display region of the display, the handle becomes an obstacle, making it difficult to expand the display region of the display.

An object of an exemplary embodiment of the invention is to provide a medical apparatus capable of expanding a display region to display more pieces of biological information.

The exemplary embodiment of the invention provides a medical apparatus that acquires biological information of a patient and that processes and displays the biological information, the medical apparatus comprising: a main body portion that includes a display region for displaying the biological information, a front portion at least partially extending in a vertical direction, a side end portion located at an end of the front portion in a lateral direction intersecting a normal direction of the front portion and intersecting the vertical direction, and a back portion located on an opposite side of the front portion in the normal direction; and a handle including a grip portion, in which the display region is provided on at least a part of the front portion, the handle is attached to the side end portion or the back portion, and an attachment position of the handle is fixed relative to the display region, and a length of the display region in a longitudinal direction is longer than a length of the display region in a width direction, and a lower end of the display region is provided below the grip portion of the handle in the vertical direction.

It is preferable that the grip portion extend in the vertical direction. It is preferable that the front portion have a first surface portion extending in the vertical direction and a second surface portion provided to be bent or curved relative to the first surface portion, and that the display region be provided on the first surface portion and the second surface portion. It is preferable that a display region corresponding to the second surface portion in the display region have a variable angle relative to the first surface portion. It is preferable that the display region be formed of a plurality of connected display panels. It is preferable that the front portion be configured with a third surface portion extending in the vertical direction, and the display region be provided on the third surface portion.

It is preferable that the medical apparatus further comprise a plurality of casters provided below the main body portion in the vertical direction, and the grip portion be provided inside a caster region having an installation position of each of the casters as a vertex. It is preferable that the medical apparatus further comprise a plurality of casters provided below the main body portion in the vertical direction, and the grip portion be provided outside a caster region having an installation position of each of the casters as a vertex.

It is preferable that at least a part of the display region be provided outward of a first surface defined by an axis in the vertical direction and an axis in the lateral direction that pass through a center-of-gravity position of the medical apparatus. It is preferable that the medical apparatus further comprise a plurality of casters provided below the main body portion in the vertical direction, and the display region be provided outward of a second surface defined by an axis in the vertical direction and an axis in the lateral direction that pass through a center position of a caster region having an installation position of each of the casters as a vertex.

It is preferable that the medical apparatus be an ultrasound diagnostic apparatus and has a probe holding portion that is provided below the grip portion in the vertical direction and that holds a probe.

According to the exemplary embodiments of the invention, it is possible to expand a display region to display more pieces of biological information.

### BRIEF DESCRIPTION OF THE DRAWINGS

FIG. 1 is a side view of a medical apparatus.
FIG. 2 is a front view of the medical apparatus.
FIG. 3 is a side view of the medical apparatus having a second plane portion of which an angle relative to a first plane portion is variable.
FIG. 4 is a front view of the medical apparatus in which a display region is formed of a plurality of display panels.
FIG. 5 is a side view of the medical apparatus having a third plane portion.
FIG. 6 is a front view of the medical apparatus having the third plane portion.
FIG. 7 is a top view of the medical apparatus.
FIG. 8 is a top view of the medical apparatus in which an installation position of a grip portion is different from that in FIG. 7.
FIG. 9 is a side view of the medical apparatus for explaining an orientation of a display region with respect to a center-of-gravity position of the medical apparatus.
FIG. 10 is a top view of the medical apparatus for explaining the orientation of the display region with respect to the center-of-gravity position of the medical apparatus.
FIG. 11 is a side view of the medical apparatus for explaining an orientation of a display region with respect to a center position of a caster region.
FIG. 12 is a top view of the medical apparatus for explaining the orientation of the display region with respect to the center position of the caster region.

### DESCRIPTION OF THE PREFERRED EMBODIMENTS

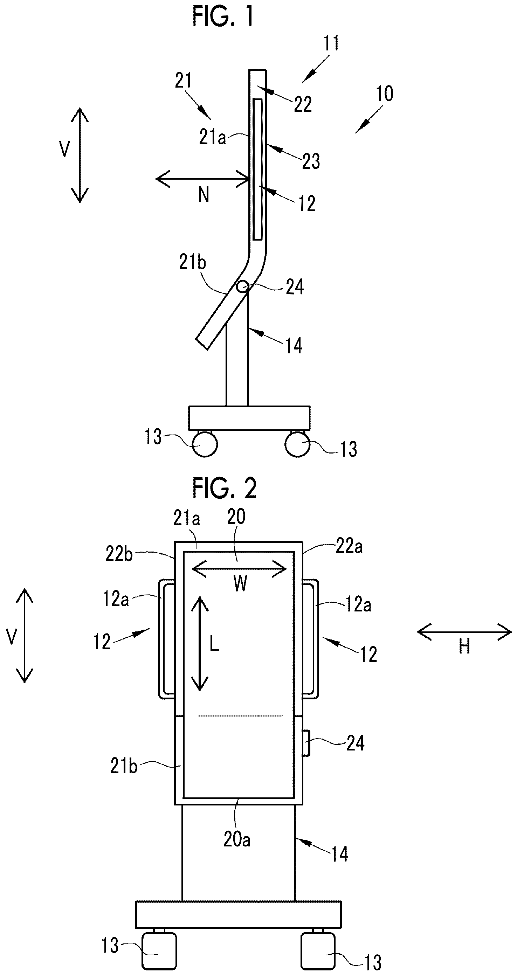

As shown in FIGS. 1 and 2, a medical apparatus 10 acquires biological information of a patient and processes and displays the biological information. Specifically, the medical apparatus 10 is an ultrasound diagnostic apparatus, and processes and displays an ultrasound image obtained by placing a probe on the patient. The medical apparatus 10 can be used as various medical apparatuses, such as a surgical apparatus, in addition to the ultrasound diagnostic apparatus.

The medical apparatus 10 has a main body portion 11, a handle 12, and casters 13. The main body portion 11 is provided on top of a base 14. The handle 12 has a grip portion 12a that is gripped by a user in a case of moving the medical apparatus 10. Four casters 13 are provided below the base 14 in a vertical direction V with respect to the main body portion 11. The entire medical apparatus 10 can be moved by these four casters 13. The number of the casters is not limited to four as long as the balance of the entire medical apparatus 10 can be maintained. In addition, the main body portion 11 is a base part of the medical apparatus 10, and does not need to be a separate body.

The main body portion 11 has a display region 20, a front portion 21, a side end portion 22, and a back portion 23. The display region 20 displays the biological information. At least a part of the front portion 21 extends in the vertical direction V. The side end portion 22 is located at an end of the front portion 21 in a lateral direction H, and has a right end portion 22a and a left end portion 22b. The lateral direction H is a direction that intersects (specifically, is orthogonal to) a normal direction N of the front portion 21 and that intersects (specifically, is orthogonal to) the vertical direction V. The back portion 23 is located on an opposite side of the front portion 21 in the normal direction N. The main body portion 11 may be provided with a controller that processes the biological information. In addition, a probe used for an ultrasound diagnosis is held by a probe holding portion 24 provided below the grip portion 12a in the vertical direction V. The front portion 21 defining the normal direction is planar rather than curved.

The display region 20 is provided on at least a part of the front portion 21, and a length of the display region 20 in a longitudinal direction L is longer than a length of the display region 20 in a width direction W. In addition, a lower end 20a of the display region 20 is provided below the grip portion 12a of the handle in the vertical direction V. In addition, the handle 12 is attached to the side end portion 22 or the back portion 23. As a result, the display region 20 extends not only near the handle 12 but also below the grip portion 12a of the handle 12 to the lower end 20a, making it possible to display more pieces of biological information. In addition, in general, the handle 12 that extends in the lateral direction H at a height of a user's waist so that the user can easily push and move the handle 12 is used. However, in the present embodiment, since the handle 12 is provided on the side end portion 22 or the back portion 23, the handle 12 does not hinder the enlargement of the display region 20 downward in the vertical direction V. It is preferable that the grip portion 12a of the handle 12 extend in the vertical direction V so that the user can easily push or pull the grip portion 12a.

Unlike the medical apparatus 10 of the present embodiment, the mobile medical apparatus in the related art has a monitor installed on an apparatus main body provided with an operation console or the like, and the monitor and the apparatus main body are often configured to move independently. This is because the orientation of the apparatus main body is adjusted by moving the casters with respect to the operation console, while, for the monitor, it is assumed that, since fine adjustment of the position and orientation of the apparatus main body is difficult, the apparatus main body is not moved each time but instead the monitor is finely adjusted in any desired direction as appropriate.

Therefore, in the mobile medical apparatus in the related art, since there is a difference in the amount of adjustment of the position and orientation between the apparatus main body and the monitor, a positional relationship between the handle and a display surface of the monitor relatively changes. On the other hand, in the present embodiment, as described above, since the lower end 20a of the display region 20 is located below the grip portion 12a of the handle in the vertical direction V and the handle 12 is attached to the side end portion 22 or the back portion 23, the attachment position of the handle 12 is fixed relative to the display region 20, thereby preventing the handle 12 from interfering with the display region 20 on the front side. Accordingly, it is possible to provide the user with the display content in the widely expanded display region without the presence of the handle 12 being an obstacle. In the mobile medical apparatus in the related art, in order to prevent the display surface of the monitor and the handle on the apparatus main body side from interfering with each other, the handle is provided below the monitor on the apparatus main body side where the operation console is provided, rather than on the monitor side, as in the medical apparatus 10 of the present embodiment.

It is preferable that the front portion 21 have a first plane portion 21a (first surface portion) extending in the vertical direction V and a second plane portion 21b (second surface portion) provided to be bent or curved relative to the first plane portion 21a, and that the display region 20 be provided along the first plane portion 21a and the second plane portion 21b. It is preferable that, in a case where the display region 20 is to be able to display the biological information as well as allow the user to perform touch operations, the biological information be displayed on a portion of the first plane portion 21a in the display region 20 and a touch operation screen is displayed on a portion of the second plane portion 21b. As a result, the user can perform touch operations at a low position by the user, such as in a case where the user sits on a chair and performs an examination or a diagnosis. The portion of the first plane portion 21a may be used a touch operation screen, and the biological information may be displayed on the portion of the second plane portion 21b, or both the display of the touch operation screen and the display of the biological information may be performed on the portion of the first plane portion 21a or on the second plane portion 21b.

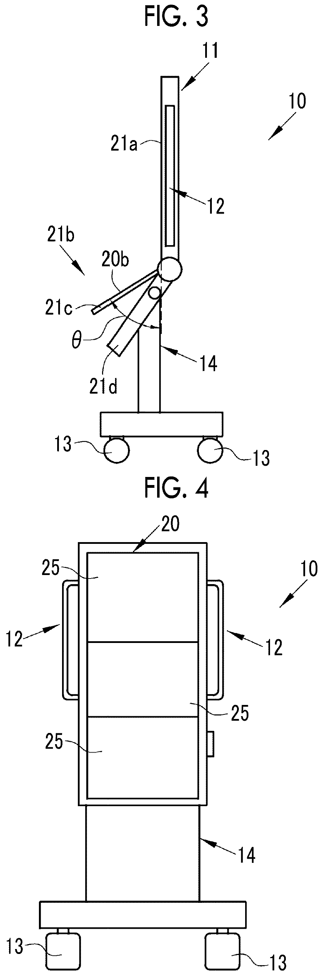

As shown in FIG. 3, it is preferable that the display region 20b corresponding to the second plane portion 21b in the display region 20 have a variable angle θ relative to the first plane portion 21a. As a result, even in a case where the biological information is displayed in a lower portion of the display region 20 as in the display region 20b corresponding to the second plane portion 21b, the biological information can be displayed according to the posture and the line of sight of the user. Specifically, it is preferable that, in order to make the angle of the display region 20b variable relative to the first plane portion 21a, the second plane portion 21b have the display region 20b, an angle variable portion 21c that can change the angle relative to the first plane portion 21a, and an angle fixed portion 21d that fixes the angle relative to the first plane portion 21a. The angle variable portion 21c is attached to the angle fixed portion 21d by a hinge (not shown) or the like.

In addition, as shown in FIG. 4, it is preferable that the display region 20 be formed of a plurality of connected display panels 25. It is preferable to connect the plurality of display panels 25 together to virtually form one panel. In this case, the biological information is displayed continuously as one virtual panel, and the touch operation is also recognized as one panel. Specifically, it is preferable that the display panels 25 be configured of three display panels 25, and it is preferable that two of the three display panels 25 be provided along the vertical direction V on the first plane portion 21a and one display panel 25 be provided on the second plane portion 21b. The number of the display panels 25 may be two or four or more.

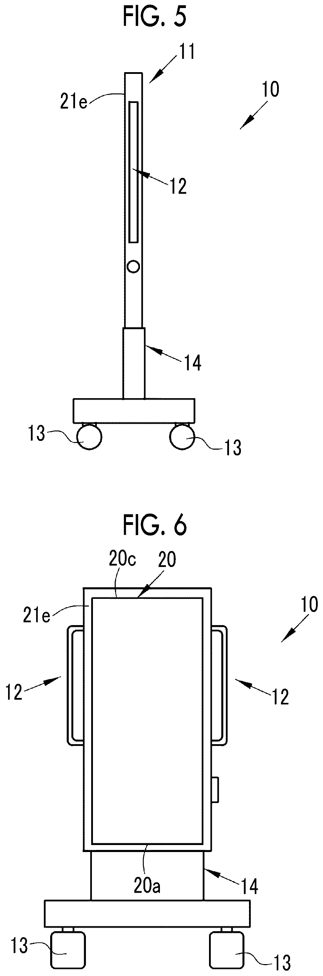

In addition, as shown in FIGS. 5 and 6, it is preferable that the front portion 21 be configured with a third plane portion 21e (third surface portion) extending in the vertical direction, and that the display region 20 be provided along the third plane portion 21e. The display region 20 provided on the third plane portion 21e is provided from an upper end 20c to the lower end 20a along the vertical direction V and is not curved or bent midway as shown in FIG. 3.

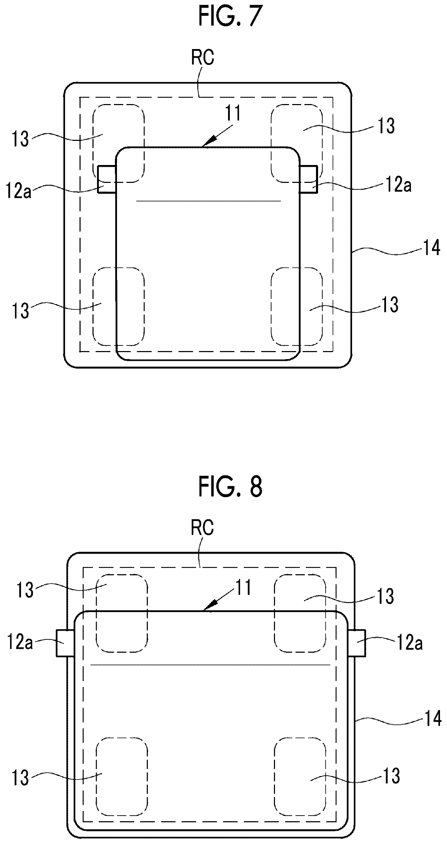

In addition, as shown in FIG. 7, it is preferable that the grip portion 12a of the handle 12 be provided inside a caster region RC having an installation position of each caster 13 as a vertex. In a case where the casters 13 are provided at four corners of the base 14, the caster region RC has a rectangular shape. As described above, by providing the grip portion 12a inside the caster region RC, it is possible to ensure stability due to the long length of the display region in the vertical direction V and to prevent the user from unintentionally coming into contact with the handle 12. In a case where the medical apparatus 10 is seldom moved and is used with its position fixed, it is preferable to adopt the installation aspect of the grip portion 12a as shown in FIG. 7.

On the other hand, as shown in FIG. 8, it is preferable that the grip portion 12a of the handle 12 be provided outside the caster region RC having the installation position of each caster 13 as the vertex. In this way, by providing the grip portion 12a outside the caster region RC, it is possible to ensure accessibility to the handle 12. This makes it easier to apply a pushing force or a pulling force to the medical apparatus 10 in a case of moving the medical apparatus 10, making it easier to move the medical apparatus 10. In a case where the medical apparatus 10 is frequently moved, it is preferable to adopt the installation aspect of the grip portion 12a as shown in FIG. 8.

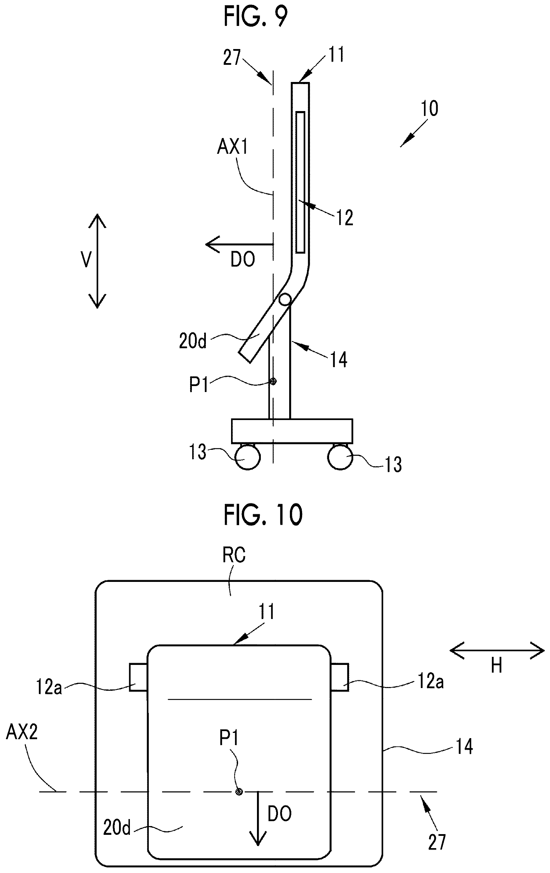

In addition, as shown in FIGS. 9 and 10, at least a part of the display region 20 is provided in an outward direction DO relative to a first plane 27 (first surface) defined by an axis AX1 in the vertical direction V and an axis AX2 in the lateral direction H that pass through a center-of-gravity position P1 of the medical apparatus 10. A partial region 20d of the display region 20 is oriented in the outward direction DO of the first plane 27. In this way, by orienting even a part of the display region 20, such as the partial region 20d, toward the outward direction DO facing the user, the display region 20 is positioned, as much as possible, on the front side where the user faces the display region 20. Accordingly, in a case where the user performs touch operations on the display region 20, the user does not need to stretch his/her arm excessively even in a case where the base 14 including the casters 13 or the like is present.

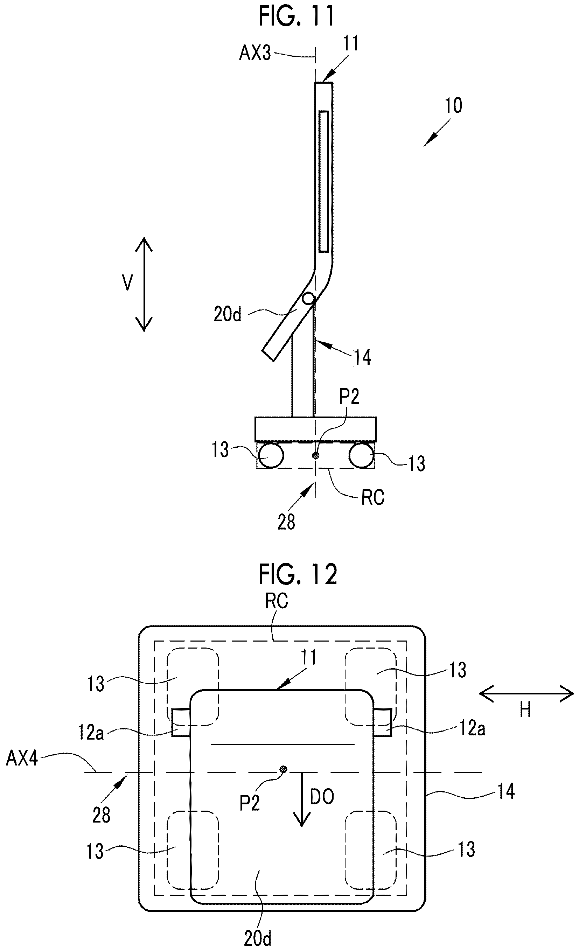

In addition, as shown in FIGS. 11 and 12, at least a part of the display region 20 is provided in an outward direction DO relative to a second plane 28 (second surface) defined by an axis AX3 in the vertical direction V and an axis AX4 in the lateral direction H that pass through a center position P2 of the caster region RC. A partial region 20d of the display region 20 is oriented in the outward direction DO of the second plane 28. In this way, by orienting even a part of the display region 20, such as the partial region 20d, toward the outward direction DO facing the user, the display region 20 is positioned, as much as possible, on the front side where the user faces the display region 20. Accordingly, in a case where the user performs touch operations on the display region 20, the user does not need to stretch his/her arm excessively even in a case where the base 14 including the casters 13 or the like is present.

In the above embodiment, the medical apparatus 10 may be configured such that the height of the main body portion 11 having the display region 20 is variable (extendable). In addition, the medical apparatus 10 may be wirelessly powered using a battery to facilitate mobility. In this case, it is preferable that the battery be disposed below the medical apparatus 10, for example, in the base 14 to improve stability. In addition, it is preferable that a status display unit that displays a status related to the biological information be provided at the uppermost portion of the display region 20. In addition, it is preferable that the medical apparatus 10 be configured to be able to change the orientation of the display region 20 by making the main body portion 11 rotatable with the vertical direction V as a rotation axis. In addition, it is preferable that the length of the main body portion 11 in the width direction W (or the distance between the casters 13) be slightly smaller than a human shoulder width so that the apparatus can be passed through a space where a person can pass during movement (for example, about 300 mm).

### Explanation of References

10: medical apparatus
11: main body portion
12: handle
12a: grip portion
13: caster
14: base
20: display region
20a: lower end
20b: display region
20c: upper end
20d: partial region
21: front portion
21a: first plane portion
21b: second plane portion
21c: angle variable portion
21d: angle fixed portion
21e: third plane portion
22: side end portion
23: back portion
24: probe holding portion
25: display panel
27: first plane
28: second plane
V: vertical direction
N: normal direction
H: lateral direction
L: longitudinal direction
W: width direction
θ: angle
RC: caster region
AX1 to AX4: axis
DO: outward direction
P1: center-of-gravity position
P2: center position

## Claims

1. A medical apparatus that acquires biological information of a patient and that processes and displays the biological information, the medical apparatus comprising:
a handle including a grip portion; and
a main body portion that includes:
a display region for displaying the biological information;
a front portion at least partially extending in a vertical direction;
a side end portion located at an end of the front portion in a lateral direction intersecting a normal direction of the front portion and intersecting the vertical direction; and
a back portion located on an opposite side of the front portion in the normal direction,
wherein the display region is provided on at least a part of the front portion,
the handle is attached to the side end portion or the back portion, and an attachment position of the handle is fixed relative to the display region, and
a length of the display region in a longitudinal direction is longer than a length of the display region in a width direction, and a lower end of the display region is provided below the grip portion of the handle in the vertical direction.

2. The medical apparatus according to claim 1,
wherein the grip portion extends in the vertical direction.

3. The medical apparatus according to either claim 1 or claim 2,
wherein the front portion has a first surface portion extending in the vertical direction and a second surface portion provided to be bent or curved relative to the first surface portion, and
the display region is provided on the first surface portion and the second surface portion.

4. The medical apparatus according to claim 3,
wherein a display region corresponding to the second surface portion in the display region has a variable angle relative to the first surface portion.

5. The medical apparatus according to any one of the preceding claims,
wherein the display region is formed of a plurality of connected display panels.

6. The medical apparatus according to any one of the preceding claims,
wherein the front portion is configured with a third surface portion extending in the vertical direction, and
the display region is provided on the third surface portion.

7. The medical apparatus according to any one of the preceding claims, further comprising:
a plurality of casters provided below the main body portion in the vertical direction,
wherein the grip portion is provided inside a caster region having installation positions of the respective casters as vertices.

8. The medical apparatus according to any one of the preceding claims, further comprising:
a plurality of casters provided below the main body portion in the vertical direction,
wherein the grip portion is provided outside a caster region having installation positions of the respective casters as vertices.

9. The medical apparatus according to any one of the preceding claims,
wherein at least a part of the display region is provided outward of a first surface defined by an axis in the vertical direction and an axis in the lateral direction that pass through a center-of-gravity position of the medical apparatus.

10. The medical apparatus according to any one of the preceding claims, further comprising:
a plurality of casters provided below the main body portion in the vertical direction,
wherein the display region is provided outward of a second surface defined by an axis in the vertical direction and an axis in the lateral direction that pass through a center position of a caster region having installation positions of the respective casters as vertices.

11. The medical apparatus according to any one of the preceding claims,
wherein the medical apparatus is an ultrasound diagnostic apparatus and has a probe holding portion that is provided below the grip portion in the vertical direction and that holds a probe.
